# EUROPÄISCHE PATENTSCHRIFT

(11) **EP 3 741 287 B1**
(45) Veröffentlichungstag und Bekanntmachung des Hinweises auf die Patenterteilung: **28.06.2023**
(21) Anmeldenummer: 20173360.7
(22) Anmeldetag: 07.05.2020
(51) Int. Cl.: A61B 1/00, A61B 1/04, G02B 23/24

(54) **ENDOSKOPKOPPLER FÜR EINE KAMERA UND VERFAHREN ZUR KOPPLUNG UND ENTKOPPLUNG EINES ENDOSKOPS MIT EINER KAMERA MITTELS EINES ENDOSKOPKOPPLERS SOWIE KAMERA MIT ENDOSKOPKOPPLER**
ENDOSCOPE COUPLER FOR A CAMERA AND METHOD FOR COUPLING AND UNCOUPLING AN ENDOSCOPE WITH A CAMERA USING AN ENDOSCOPE COUPLER AND CAMERA WITH ENDOSCOPE COUPLER
COUPLEUR D'ENDOSCOPE POUR UNE CAMÉRA ET PROCÉDÉ DE COUPLAGE ET DE DÉCOUPLAGE D'UN ENDOSCOPE DOTÉ D'UNE CAMÉRA AU MOYEN D'UN COUPLEUR D'ENDOSCOPE AINSI QUE CAMÉRA POURVUE DE COUPLEUR D'ENDOSCOPE

(30) Priorität: 21.05.2019 DE 102019113435
(43) Veröffentlichungstag der Anmeldung: 25.11.2020
(73) Patentinhaber: ATMOS MedizinTechnik GmbH & Co. KG, 79853 Lenzkirch (DE)
(72) Erfinder: Boysen, Björn, 79853 Lenzkirch (DE); Reinhardt, Carsten, 79853 Lenzkirch (DE)
(74) Vertreter: Westphal, Mussgnug & Partner Patentanwälte mbB

(56) Entgegenhaltungen:
- DE-A1- 2 346 197
- DE-A1- 4 444 049
- DE-C1- 19 712 645
- DE-U1- 7 918 414

## Beschreibung

Endoskopische Verfahren stellen eine der wichtigsten Methoden dar, um das Innere des menschlichen Körpers -insbesondere visuell- zu untersuchen und gegebenenfalls eine Behandlung einzuleiten. Zur Dokumentation der dabei erhaltenen Befunde, aber auch einer etwaigen Behandlung kommen dabei Kameras zum Einsatz, die mittels eines Endoskopkopplers mit dem proximalen Endbereich des Endoskops mit einem Haltemechanismus lösbar verbunden werden.

Derartige Endoskopkoppler sind bereits aus dem Stand der Technik bekannt, beispielsweise aus der DE 10 2012 005 037 A1, der WO 2013/156024 A1 oder der DE 10 2014 222 880 A1. Nachteilig bei den bekannten Endoskopkopplern ist jedoch, dass der Haltemechanismus zur lösbaren Verbindung einen vergleichsweise hohen Bedienaufwand erfordert, weil jeweils eine Fixierungsmimik gelöst werden muss und insbesondere eine einhändige Bedienung nicht oder nur unter großen Schwierigkeiten möglich ist. Ein Endoskopkoppler mit den Merkmalen des Oberbegriffs des Anspruchs 1 ist aus der DE 44 44 049 A1 bekannt.

Die Aufgabe der Erfindung besteht daher darin, einen mit weniger Bedienaufwand zu bedienenden Endoskopkoppler und jeweils ein Verfahren zur Kopplung eines Endoskops mit einer Kamera mittels eines Endoskopkopplers bzw. zur Entkopplung eines Endoskops von einer Kamera mittels eines Endoskopkopplers bereitzustellen, das die Kopplung bzw. Entkopplung vereinfacht. Ferner besteht eine Aufgabe der Erfindung darin, eine Kamera bereitzustellen, welche einen solchen mit weniger Bedienaufwand zu bedienenden Endoskopkoppler aufweist.

Diese Aufgabe wird gelöst durch einen Endoskopkoppler mit den Merkmalen des Patentanspruchs 1, und durch ein Verfahren mit den Merkmalen des Patentanspruchs 9 bzw. ein Verfahren mit den Merkmalen des Patentanspruchs 10 sowie durch eine Kamera mit den Merkmalen des Patentanspruchs 12. Vorteilhafte Weiterbildungen des Endoskopkopplers beziehungsweise des Verfahrens sind Gegenstand der abhängigen Ansprüche.

Der erfindungsgemäße Endoskopkoppler für eine Kamera hat ein proximales Ende zur Anordnung an der Kamera, einen Kamerabefestigungsmechanismus zur Befestigung des Endoskopkopplers an der Kamera und eine Endoskopkopfaufnahmebereich zur zumindest teilweisen Aufnahme eines Endoskopkopfes wenn ein Endoskop mit der Kamera gekoppelt ist, wobei der Endoskopkopfaufnahmebereich einen Zugang für den Endoskopkopf aus Richtung des distalen Endes des Endoskopkopplers aufweist. Ferner hat der Endoskopkoppler einen Endoskopfixiermechanismus zur Befestigung des Endoskops an dem Endoskopkoppler.

Der Kamerabefestigungsmechanismus kann beispielsweise eine übliche Schraubverbindung, ein Bajonett-Verschluss, ein Abschnitt, der mit der Kamera verpresst wird oder ist oder ein Click-Verschluss sein.

Erfindungswesentlich ist, dass der Endoskopfixiermechanismus mindestens eine beweglich gelagerte Backe aufweist, die mit einer Federkraft derart beaufschlagt ist, dass zumindest ein Bereich der Backe in den Zugang hineingedrückt ist und den Zugang sperrt, wobei zumindest der in den Zugang hineinragende Bereich der Backe so geformt ist, dass sich sein distaler Abschnitt in Richtung auf das distale Ende des Endoskopkopplers hin verjüngt, so dass ein Druck auf den distalen Abschnitt des in den Zugang hineinragenden Bereichs der Backe in Richtung auf das proximale Ende des Endoskopkopplers eine der Federkraft entgegenwirkende Kraft erzeugt.

Auf diese Weise wird es möglich, den Endoskopkopf, insbesondere einen Okulartrichter des Endoskopkopfes, der vorzugsweise der DIN 58105 entspricht, einfach in Richtung auf das proximale Ende des Endoskopkopplers gegen die so ausgestalteten Backen zu drücken.

Unmittelbar zu Beginn des Kontakts kann insbesondere dann, wenn mehr als eine solche Backe vorhanden ist, was bevorzugt ist -besonders bevorzugt ist eine Ausführungsform mit mindestens drei solchen Backen- und wenn die Backen hinsichtlich ihrer Geometrie identisch ausgeführt sind, eine Selbstzentrierung des Endoskopkopfes erreicht werden.

Danach wird wegen des sich verjüngenden Abschnitts der Backe beim Andruck eine Kraftkomponente erzeugt, die der Federkraft in radialer Richtung entgegenwirkt und sie schließlich überwindet, so dass die Backe immer weiter aus dem Zugang zum Endoskopkopfaufnahmebereich hinausgedrückt wird und sich der Endoskopkopf immer weiter in Richtung auf den Endoskopkopfaufnahmebereich bewegt, bis die Backe den Zugang komplett freigibt und der Endoskopkopf in den Endoskopkopfaufnahmebereich eintritt.

Da der Endoskopkopf bzw. sein Okulartrichter sich in distaler Richtung verjüngt, beginnt sich beim weiteren Vordringen des Endoskopkopfes in den Endoskopkopfaufnahmebereich die Backe wieder in den Zugang hineinzuschieben und die Bewegung des Endoskopkopfes in distaler Richtung immer weiter zu sperren, bis schließlich der Endoskopkopf am Boden des Endoskopkopfaufnahmebereichs anschlägt.

In diesem Zustand kann -in Abhängigkeit insbesondere von der Federkraft, mit der die Backe beaufschlagt wird- allenfalls noch eine Rotation des Endoskopkopfs relativ zum Endoskopkoppler erfolgen; der Endoskopkopf ist insoweit zumindest gegen eine Verschiebung fixiert.

Erfindungswesentlich ist es, wenn der Endoskopkoppler auch einen Lösemechanismus aufweist, bei dessen Betätigung in den Zugang hineinragende Bereiche der Backe aus dem Zugang herausbewegt werden oder aus dem Zugang herausbewegbar werden. Dies wird dadurch erreicht werden, dass der Lösemechanismus derart aufgebaut ist, dass er bei Betätigung die Federkraftbeaufschlagung der Backe reduziert.

Vorzugsweise ist dabei der Lösemechanismus so aufgebaut, dass er durch eine Drehung in Umfangsrichtung des Endoskopkopplers ausgelöst wird. Eine solche Drehung lässt sich leicht mit einer Hand ausführen und kann dann alle Backen gleichzeitig lösen, während Lösemechanismen, die auf eine Translationsbewegung setzen, im Allgemeinen lediglich das Lösen einer einzelnen Backe herbeiführen können.

Wenn zudem zumindest der in den Zugang hineinragende Bereich der Backe so geformt ist, dass sich sein proximaler Abschnitt in Richtung auf das proximale Ende des Endoskopkopplers hin schneller verjüngt als sich der distale Abschnitt in Richtung auf das distale Ende des Endoskopkopplers hin verjüngt wird einerseits das Fixieren des Endoskopkopfes, insbesondere eines Okulartrichters, wenn er in den Endoskopkopfaufnahmebereich eingeführt ist, verbessert.

Andererseits kann man so erreichen, dass der Löseprozess ebenfalls sehr kontrolliert abläuft und bei einer lediglich versehentlichen Betätigung des Lösemechanismus nicht sofort eintritt, sondern erst dann, wenn ein zusätzlicher Zug in distaler auf den Endoskopkkopf ausgeübt wird. Das Wirkungsprinzip ist dabei ähnlich wie beim Einführen des Endoskopkopfes, der Unterschied in den Steigungen bei der Verjüngung führt aber dazu, dass sehr viel höhere Zugkräfte nötig wären, um die Backe aus dem Zugang zum Endoskopkopfaufnahmebereich hinauszudrücken wenn nicht die Federkraftbeaufschlagung der Backe durch Betätigung des Lösemechanismus reduziert wird. Der Lösemechanismus kann aber so ausgelegt werden, dass noch eine Restfederkraftbeaufschlagung der Backe bleibt, die aktiv durch Zug überwunden werden muss.

Gemäß einer bevorzugten Weiterbildung der Erfindung ist die beweglich gelagerte Backe auf ihrer einen Seite um eine parallel zur Einführrichtung des Endoskopkopfes verlaufende Achse drehbar gelagert. Besonderes bevorzugt ist es dabei, wenn diese Achse ihrerseits in einem insbesondere ringförmigen Bauteil gelagert ist, das in Umfangsrichtung gemeinsam mit der Backe drehbar ist. Beispielsweise durch Zusammenwirken mit einer geeigneten Mimik oder Kulisse kann dann auf besonders einfache Weise ein Lösemechanismus realisiert werden, der durch Drehung und bevorzugt einhändig auslösbar ist.

Um eine solche Drehbarkeit auch der gelagerten Achse mit der daran angeordneten Backe in Umfangsrichtung zu ermöglichen ist es vorteilhaft, wenn die um eine parallel zur Einführrichtung des Endoskopkopfes verlaufende Achse drehbare Lagerung durch einen ersten Stift erfolgt, der sich entweder nur in Richtung auf das distale Ende des Endoskopkopplers hin oder nur in Richtung auf das proximale Ende des Endoskopkopplers hin über die Backe hinausragt.

Eine Führung, mit der der Eingriff der Backe in den Zugang kontrolliert werden kann, kann insbesondere dadurch realisiert werden, dass die beweglich gelagerte Backe auf ihrer anderen Seite in einer Führungsnut verschiebbar gelagert ist. Besonders einfach kann man eine solche Art der Führung realisieren, wenn die in der Führungsnut verschiebbare Lagerung durch einen zweiten Stift erfolgt, der nur in die der Richtung, in der der erste Stift, durch den die parallel zur Einführrichtung des

Endoskopkopfes verlaufende Achse drehbare Lagerung erfolgt, über die Backe hinausragt, gegenüberliegende Richtung über die Backe hinausragt.

Eine besonders vorteilhafte Möglichkeit der Realisierung der Druckbeaufschlagung mittels einer Federkraft besteht dann darin, dass die Federkraftbeaufschlagung durch eine in der Führungsnut angeordnete Feder, insbesondere bevorzugt eine Druckfeder, erfolgt. Insbesondere kann in dieser Konfiguration auch erreicht werden, dass der Lösemechanismus nach Betätigung automatisch wieder in die Verriegelungsposition zurückgeführt wird.

Das erfindungsgemäße Verfahren zur Kopplung eines Endoskops an eine Kamera unter Verwendung eines Endoskopkopplers weist die Schritte Befestigen des Endoskopkopplers an der Kamera und Befestigen des Endoskopkopfs an dem Endoskopkoppler und zeichnet sich dadurch aus, dass die Kopplung des Endoskopkopfes an den Endoskopkoppler lediglich durch Ausüben von Druck erfolgt. Damit wird eine extrem einfache Kopplungsmöglichkeit geschaffen, die auch einhändig ausgeführt werden kann.

Erfindungswesentlich ist dabei ein Verfahren, bei dem als Endoskopkoppler ein erfindungsgemäßer Endoskopkoppler, bevorzugt gemäß einer der oben beschriebenen Weiterbildungen, verwendet wird.

Das erfindungsgemäße Verfahren zur Entkopplung eines Endoskops von einem Endoskopkoppler zeichnet sich dadurch aus, dass das Verfahren das Betätigen von genau einem Lösemechanismus umfasst, der auf sämtliche Fixiermittel, die das Endoskop am Endoskopkoppler halten, wirkt. Dadurch wird ein einfacher, insbesondere im Regelfall auch nur mit einer Hand ausführbarer Entkopplungsprozess ermöglicht.

Erfindungswesentlich

ist es dabei, wenn als Endoskopkoppler ein erfindungsgemäßer Endoskopkoppler mit einem Lösemechanismus verwendet wird, wobei vorzugsweise der Lösemechanismus durch eine Drehbewegung in Umfangsrichtung des Endoskopkopplers betätigt wird. Die erfindungsgemäße Kamera zeichnet sich durch einen erfinudngsgemäßen Endoskopkoppler aus. In einer bevorzugten Weiterbildung der Erfindung ist der Endoskopkoppler (1) mit der Kamera fest verbunden, insbesondere verpresst und/oder verschraubt.

Besonders bevorzugt ist dabei, wenn ein Teil des optischen Systems der Kamera am Endoskopkoppler angeordnet ist. Dadurch ist eine besonders effiziente Bauraumnutzung möglich.

Die Erfindung wird nachfolgend anhand von Figuren, die Ausführungsbeispiele der Erfindung zeigen, näher erläutert. Es zeigt:
- Fig. 1a:: Eine Explosionsdarstellung eines Endoskopkopplers,
- Fig. 1b:: eine Aufsicht auf den Endoskopkoppler aus Figur 1a,
- Fig. 1c:: einen ersten Querschnitt durch den Endoskopkoppler aus Figur 1b längs der Schnittlinie A-A,
- Fig. 1d:: einen zweiten Querschnitt durch den Endoskopkoppler aus Figur 1b längs der Schnittlinie B-B,
- Fig. 1e:: einen dritten Querschnitt durch den Endoskopkoppler aus Figur 1b längs der Schnittlinie C-C,
- Fig. 2:: eine schematische Darstellung des Ausgangszustands beim Koppeln eines Endoskopkopplers und eines Endoskopkopfes eines mit dem Endoskopkoppler zu koppelnden Endoskops vor dem Koppeln,
- Fig. 3a:: eine Ansicht des Endoskopkopplers und Endoskopkopfes aus Figur 2 unmittelbar vor dem Koppeln,
- Fig. 3b:: eine zweite Ansicht des Endoskopkopplers aus Figur 3a, jedoch ohne Endoskopkopf und ohne Abdeckring, vor dem Koppeln,
- Fig. 4a:: eine Ansicht des Endoskopkopplers und Endoskopkopfes aus Figur 2 beim Koppeln,
- Fig. 4b:: eine zweite Ansicht des Endoskopkopplers aus Figur 4a jedoch ohne Endoskopkopf und ohne Abdeckring, beim Koppeln,
- Fig. 5a:: eine Ansicht des Endoskopkopplers und Endoskopkopfes aus Figur 3 im gekoppelten Zustand, und
- Fig. 5b:: eine zweite Ansicht des Endoskopkopplers aus Figur 5a jedoch ohne Endoskopkopf und ohne Abdeckring, im gekoppelten Zustand.

Gleiche Bestandteile gleicher Ausführungsformen des Endoskopkopplers sind in den Figuren jeweils mit den gleichen Bezugszeichen versehen, soweit nichts anderes erwähnt ist. Allerdings sind zur Steigerung der Übersichtlichkeit nicht in allen Figuren sämtliche Bezugszeichen eingetragen.

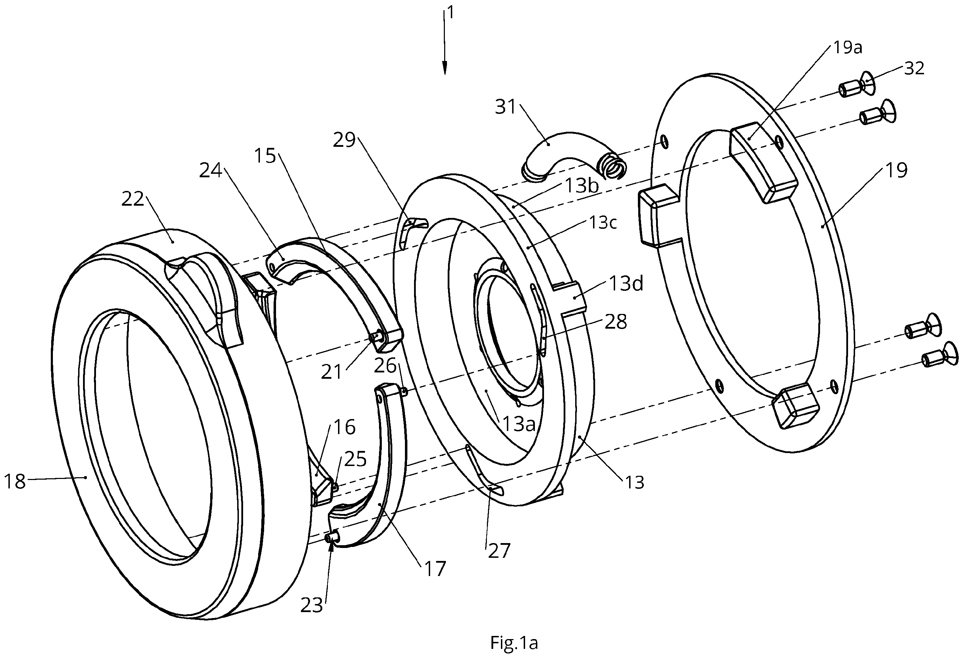

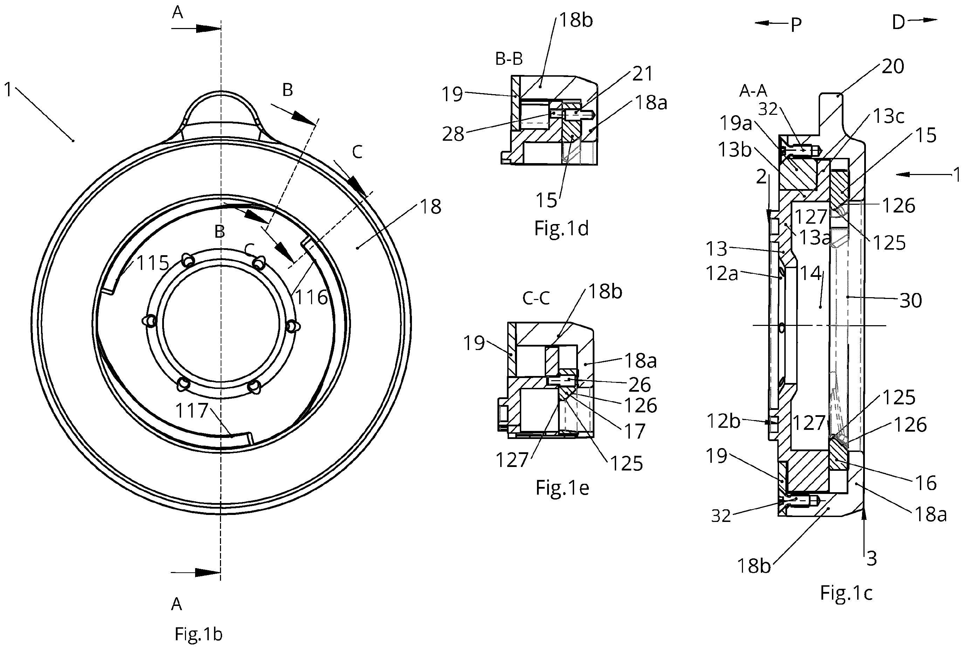

Die Figuren 1a bis 1e zeigen einen Endoskopkoppler 1 mit Backen 15,16,17. Insbesondere zeigt Figur 1a eine Explosionsdarstellung, Figur 1b eine Aufsicht auf den Endoskopkoppler betrachtet in Einschubrichtung eines zu koppelnden Endoskopkopfes, Figur 1c einen Querschnitt durch einen Endoskopkoppler 1 längs der Linie A-A, die einem Durchmesser des Endoskopkopplers 1 entspricht, Figur 1d einen halbseitigen Querschnitt durch den Endoskopkoppler 1 längs der Linie B-B, die eine Schnittebene durch ein erstes der Lager der Backen 15,16,17 am Beispiel der Backe 15 definiert und Figur 1e einen halbseitigen Querschnitt durch den Endoskopkoppler 1 längs der Linie C-C, die eine Schnittebene durch ein zweites der Lager der Backen 15,16,17 am Beispiel der Backe 16 definiert.

Wie man der Zusammenschau dieser Figuren entnimmt, hat der Endoskopkoppler 1, wie in Figur 1c besonders gut zu erkennen ist, ein proximales Ende 2 zur Anordnung des Endoskopkopplers 1 an einer nicht gezeigten Kamera und mit einem dem proximalen Ende 2 gegenüberliegenden distalen Ende 3. Daraus ergibt sich unmittelbar die Definition der jeweils zur Veranschaulichung als Pfeil eingetragenen proximalen Richtung P und distalen Richtung D für den Endoskoppkoppler 1.

Der Endoskopkoppler 1 weist, wie die Zusammenschau der Figuren 1a und 1c besonders deutlich zeigt, eine topfförmige Endoskopaufnahme 13 mit Boden 13a, umlaufender Seitenwand 13b und einem breiten, parallel zum Boden 13a verlaufenden Randbereich 13c auf, der sich vom distalen Ende des Randbereichs in radialer Richtung nach außen erstreckt. Ferner weist die topfförmige Endoskopaufnahme 13 Lagerabschnitte 13d auf, die in diesem Beispiel als lokale, blockartig radial nach außen vorspringende Verdickungen des Randbereichs 13c ausgeführt sind

Der Innenraum der topfförmigen Endoskopaufnahme 13 bildet den Endoskopkopfaufnahmebereich 14. Am Boden 13a der topfförmigen Endoskopaufnahme 13 ist eine Öffnung vorhanden, durch die das Bild vom Endoskop zur Kamera gelangen kann; optional können hier auch optische Elemente, z.B. ein Linsensystem, angeordnet werden.

Ferner sind in diesem Ausführungsbeispiels am Boden 13a ein Kamerabefestigungsmechanismus 12a, der Öffnungen zum Verschrauben des Endoskopkopplers 1 mit der Kamera aufweist und ein Kamerabefestigungsmechanismus 12b zum Verpressen mit eine Rand eines Objektivs der Kamera angeformt. Grundsätzlich kann der Kamerabefestigungsmechanismus aber auch mit einer separaten, vorzugsweise kreisringförmigen Bodenplatte verbunden sein, die ihrerseits dann die topfförmige Endoskopaufnahme 13 trägt.

Wie die Figuren 1c,1d und 1e besonders deutlich zeigen, wird der Randbereich 13c der Endoskopaufnahme 13 wird von einer Baugruppe umgriffen, die in diesem Ausführungsbeispiel aus Backen 15,16,17, die auf dem Randbereich 13c aufliegen, einem Vorsatz 18 mit kreisringförmigem Deckel 18a und einem sich senkrecht vom Außenrand des Deckels 18a in proximaler Richtung P über die Position des Randbereichs 13c hinaus erstreckenden umlaufenden Rand 18b, der die seitliche Außenwand des Endoskopkopplers 1 bildet sowie einem Ring 19, der mit in diesem Beispiel drei lokalen Vorsprüngen 19a, welche sich in distaler Richtung D erstrecken und an der Unterseite des Randbereichs 13c anliegen, in radialer Richtung an der Außenseite der umlaufenden Seitenwand 13b anliegt gebildet wird. Mit dieser Baugruppe ist ferner ein Bedienelement 20 verbunden, was im gezeigten Beispiel konkret durch Anformen an den Vorsatz 18 im Übergangsbereich zwischen Deckel 18a und umlaufendem Rand 18b realisiert ist.

Dabei ist zur Bildung der Baugruppe der Ring 19 mit dem Rand 18b des Vorsatzes 18 verbunden, was in diesem Beispiel durch Schrauben 32 erfolgt, und die Backen 15,16,17 sind jeweils in ihrem Endbereich mit ersten Stiften 21,22,23, die wie z.B. in der Darstellung der Figur 1a oder 1d zu erkennen ist, parallel zur Einführrichtung E des Endoskopkopfs 90 verlaufen, mit dem kreisringförmigen Deckel 18a verbunden, wobei die Lagerung der Stifte 20,21,22 in Backen 15,16,17 und Deckel 18a jedoch so ausgeführt ist, dass eine Rotation möglich ist. Demzufolge sind die Backen 15,16,17 jeweils um eine Achse, die parallel zur Einführrichtung E des Endoskopkopfs 90 verläuft drehbar.

Die vorstehend geschilderte Baugruppe ist grundsätzlich relativ zur Endoskopaufnahme 13 verdrehbar angeordnet, das Ausmaß, in dem diese Bewegung möglich ist, ist allerdings dadurch beschränkt, dass jeweils an dem anderen Ende der Backen 15,16,17, das dem Ende, an dem die ersten Stifte 21,22,23 angeordnet sind, zweite Stifte 24,25,26, die z.B. in den Figuren 1a oder 1e erkennbar sind, vorhanden sind, die jeweils in einer auf der distalen Seite des Randabschnitts 13c eingebrachten Führungsnut 27,28,29 geführt und mit der Kraft einer Feder 31, die in den Figuren 1a sowie 3b, 4b und 5b erkennbar ist, beaufschlagt sind. Die Feder 31 ist zwischen einem Vorsprung 19a und einem Lagerabschnitt 13d eingespannt und wirkt als Druckfeder, die einer relativen Verdrehung zwischen der oben beschriebenen Baugruppe und der Endoskopaufnahme 13 entgegen wirkt.

Die Öffnung des kreisringförmigen Deckels 18a fluchtet mit dem Endoskopkopfaufnahmebereich 14 und definiert einen Zugang 30 zu diesem, der durch den Raumbereich der Öffnung zwischen der das distale Ende 2 des Endoskopkopplers 1 definierenden Oberseite des kreisringförmigen Deckels 18a und der distalen Oberseite des Randbereichs 18c gebildet wird.

Bereits die Darstellung der Figur 1b zeigt, dass die Backe 15 einen Bereich 115 aufweist, der in den Zugang 30 hineinragt und auf diese Weise sperrt. Ausgehend vom am weitesten in den Zugang 30 hineinragenden Punkt 125 schließt sich einerseits ein distaler Abschnitt 126 des in den Zugang 30 hineinragenden Bereichs 115 an, in dem sich die Backe 15 in Richtung auf das distale Ende 3 des Endoskopkopplers 1 hin verjüngt, wobei das distale Ende des Bereichs 115 mit dem Innenrand des kreisringförmigen Deckels 18a fluchtet. Andererseits ist ausgehend von diesem Punkt 125 ein proximaler Abschnitt 127 des in den Zugang 30 hineinragenden Bereichs 115 vorhanden, welcher sich in Richtung auf das proximale Ende 2 des Endoskopkopplers 1 in schneller verjüngt als sich der distale Abschnitt 126 des in den Zugang 30 hineinragenden Bereichs 115 verjüngt.

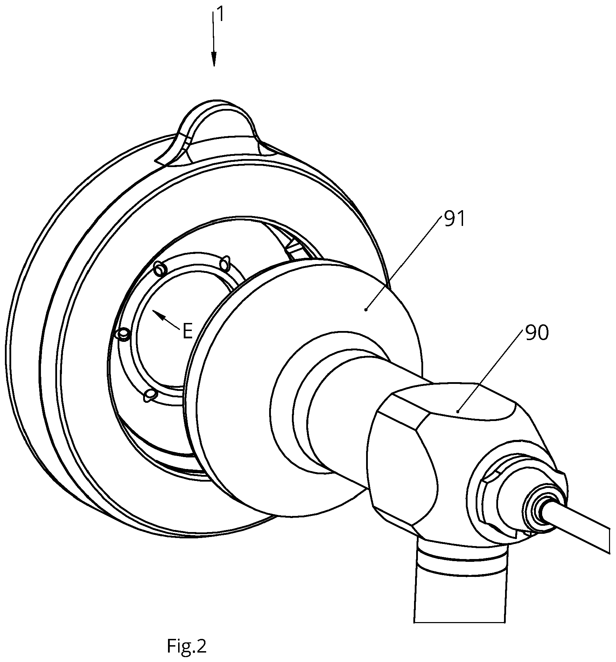

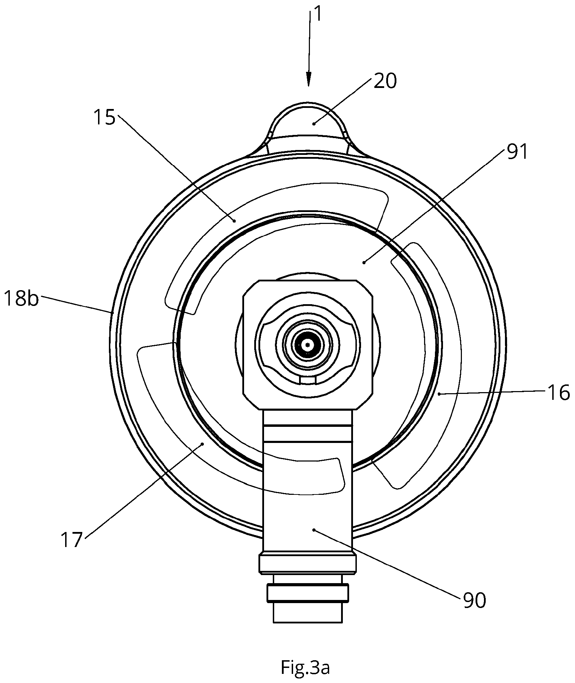

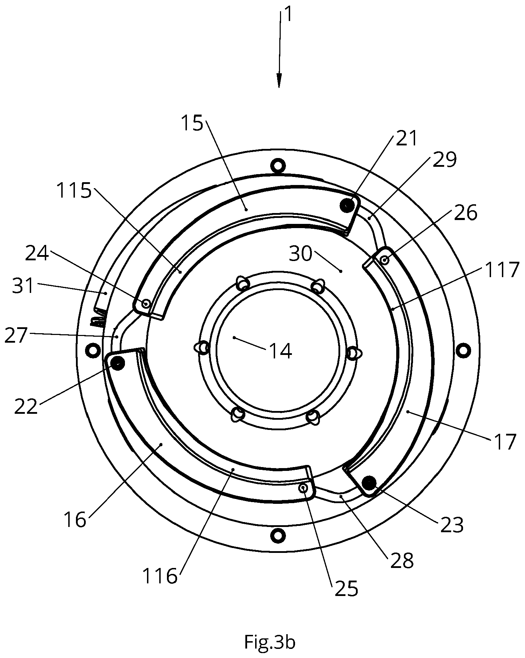

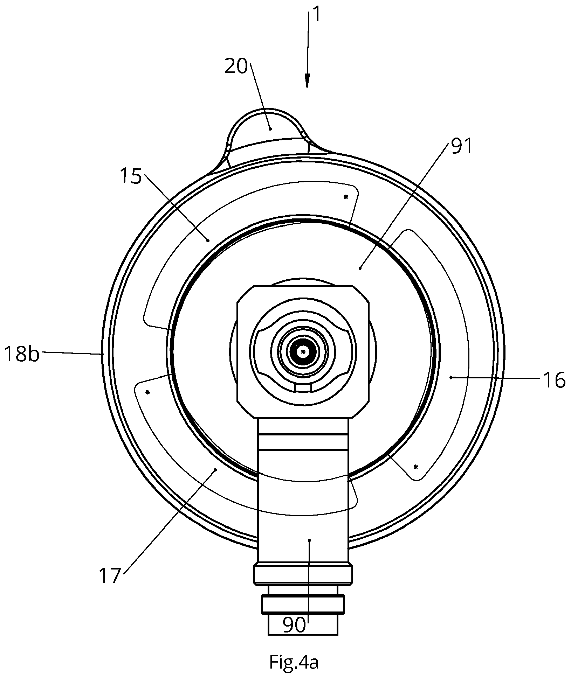

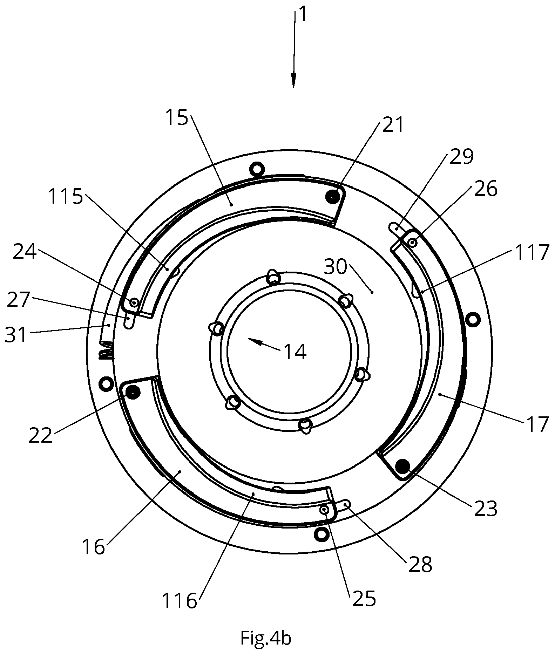

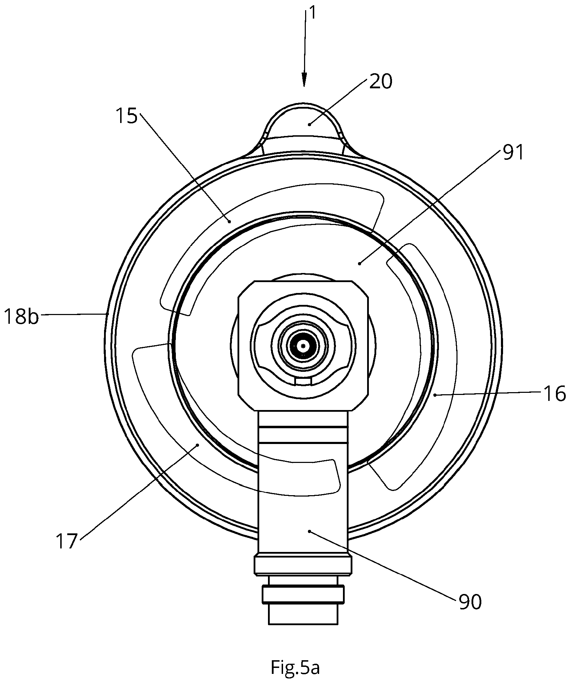

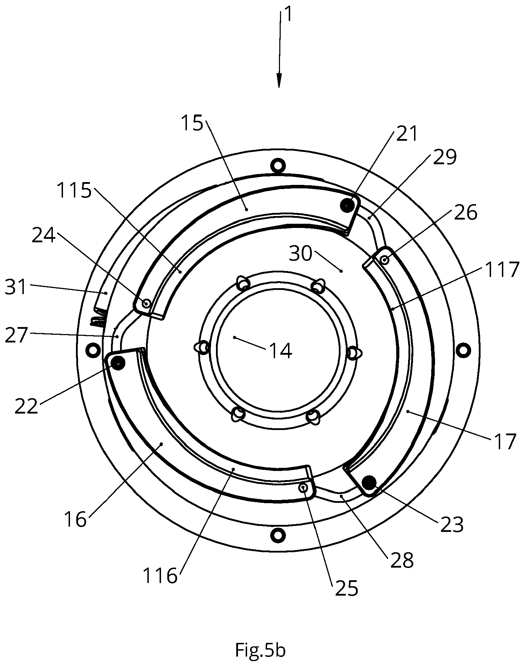

Anhand der Figuren 2 bis 5b soll nun die Wirkungsweise des Endoskopkopplers 1 detailliert dargelegt werden. Dazu ist in Figur 2 eine schematische Darstellung des Ausgangszustands beim Koppeln eines Endoskopkopplers 1 und eines Endoskopkopfs 90 eines mit dem Endoskopkoppler 1 zu koppelnden Endoskops vor dem Koppeln, inden Figuren 3a und 3b die Situation vor dem Koppeln eines Endoskopkopfs 90, in den Figuren 4a und 4b die Situation während der Endoskopkopf 90 gerade gekoppelt wird und in den Figuren 5a und 5b die Situation nach dem Koppeln des Endoskopkopfs 90 gezeigt.

Der Endoskopkoppler 1 ist in all den Figuren 3a,4a und 5a mit einem teilweise transparenten Deckel 18a und Endoskopkopf 90, so dass die Betrachtung der Position der sonst in diesen Ansochten eigentlich nicht sichtbaren Backen 15,16,17 in diesen Figuren ermöglich wird, und in den Figuren 3b,4b und 5b jeweils ohne den kreisringförmigen Deckel 18a dargestellt, um das Nachvollziehen insbesondere der Bewegung der Backen 15,16,17 durch Einblick in das Innere des Endoskopkopplers 1 zu erleichtern. Diese Konfiguration wird nachfolgend als der geöffnete Endoskopkoppler 1 bezeichnet.

Wichtig für das richtige Verständnis dieser Figuren ist dabei, in Erinnerung zu behalten, dass die Backen 15,16,17 sich mit dem umlaufenden Rand 18b, dem mit diesem verbundenen Ring 19 und dem am umlaufenden Rand 18b angeordneten Bedienelement 20 bewegen, während die topfförmige Endoskopaufnahme 13, die über die Befestigungsmittel 12a,12b mit der nicht dargestellten Kamera verbunden ist, ortsfest bleibt.

Figur 2 zeigt den geöffneten Endoskopkoppler 1 und den Endoskopkopf 90 mit Okulartrichter 91 in einer möglichen Ausgangsposition vor dem Beginn des Kopplungsprozesses. Die Einführrichtung E ist durch einen Pfeil angedeutet, sie verläuft von in proximaler Richtung des Endoskopkopplers, also auf dessen proximales Ende 2 zu.

In Figur 3b kann man besonders gut den Zustand der Backen 15,16,17 des Endoskopkopplers 1 in diesem Zustand erkennen: Durch Wechselwirkung mindestens einer Feder 31 mit den in den Führungsnuten 27,28,29 geführten zweiten Stiften 24,25,26 werden die Abschnitte 116,117,118 in den Zugang 30 hineingedrückt und sperren ihn, so dass der Okulartrichter 91 des Endoskopkopfs 90, wie in Figur 3a besonders gut zu sehen ist, nicht ohne Wechselwirkung mit den Backen 15,16,17 des Endoskopkopplers 1 in den Endoskopkopfaufnahmebereich 14 erfolgen kann.

Wird nun der Endoskopkopf 90 mit dem proximalen Rand des Okulartrichters 91 in Anlage mit den Backen 15,16,17, insbesondere deren distalen Abschnitten 126 im Bereich der in den Zugang 30 hineinragenden Bereiche 115,116,117 gebracht und Druck ausgeübt, der stark genug ist, um die Federkraft zu überwinden, weichen die Backen 15,16,17 in der jeweiligen Führungsnut 27,28,29 geführt aus. Wegen der Verbindung der Backen 15,16,17 über die ersten Stifte 21,22,23 zum nicht dargestellten kreisförmigen Deckel 18a führt dies dazu, dass sich dessen gesamte Baugruppe einschließlich des umlaufenden Randes 18b, des Rings 19 und des Bedienelements 20 dreht, während die in den Zugang 30 hineinragenden Bereiche 115,116,117 immer weiter aus dem Zugang 30 hinausgedrückt werden, bis schließlich in der in den Figuren 4a und 4b gezeigt Zustand erreicht ist, in dem der Endoskopkopf 90 mit dem Okulartrichter 91 in den Endoskopkopfaufnahmebereich 14 einzutreten beginnt. In diesem Zustand ist die Feder 31 maximal gespannt, d.h., da sie als Druckfeder konfiguriert ist, maximal zusammengedrückt.

Sobald der äußere Rand des Okulartrichters 91 den am weitesten in den Zugang hineinragenden Punkt 125 der Bereiche 115,116, 117 der Backen 15,16,17 passiert hat, gelangt er in den proximalen Abschnitt 127 der Bereiche 115,116,117 und die Backen 15,16,17 beginnen sich, getrieben von der Federkraft, wieder zu schließen, so dass die Bereiche 115,116,117 der Backen 15,16,17 wieder in den Zugang 30 einzutreten beginnen. Dadurch wird der äußere Rand des Okulartrichters 91 an den Boden 13a der Endoskopaufnahme 13 gedrückt - der Endoskopkopf 90 ist damit an den Boden 13a geklemmt und kann nicht mehr ohne weiteres den Endoskopkopfaufnahmebereich 14 verlassen, wie die Figuren 5a und 5 veranschaulichen.

Um dies am Ende der Verwendung des Endoskops dennoch zu ermöglichen kann man mit Hilfe des Betätigungselements 20 die Baugruppe mit umlaufendem Rand 18b, Ring 19, kreisringförmigem Deckel 18a und daran mittels der ersten Stifte 21,22,23 angeordneten Backen 15,16,17 entgegen der Federkraft der nicht dargestellten Feder 31 in dieselbe Richtung drehen, in die er sich beim Einführen des Endoskopkopfs 90 bedingt durch die Wechselwirkung zwischen Endoskopkopf 90 und Backen 15,16,17 bewegt. Dies erzwingt eine Bewegung der Backen 15,16,17 in der Führungsnut 27,28,29, die die Bereiche 115,116,117 aus dem Zugang 30 hinauszieht. Der Endoskopkopf 90 wird freigegeben und kann wieder entfernt werden.

### Bezugszeichenliste

- 1: Endoskopkoppler
- 2: proximales Ende
- 3: distales Ende

- 12a: Kamerabefestigungsmechanismus
- 12b: Kamerabefestigungsmechanismus
- 13: Endoskopaufnahme
- 13a: Boden
- 13b: Seitenwand
- 13c: Randbereich
- 13d: Lagerabschnitt
- 14: Endoskopkopfaufnahmebereich
- 15,16,17: Backe
- 18: Vorsatz
- 18a: Deckel
- 18b: Rand
- 19: Ring
- 19a: Vorsprung
- 20: Bedienelement
- 21,22,23: erster Stift
- 24,25,26: zweiter Stift
- 27,28,29: Führungsnut
- 30: Zugang
- 31: Feder
- 32: Schraube

- 115,116,117: Bereich
- 125: Punkt
- 126: distaler Abschnitt
- 127: proximaler Abschnitt
- P: proximale Richtung
- D: distale Richtung
- E: Einführrichtung
- A-A: Schnittlinie
- B_B: Schnittlinie
- C-C: Schnittlinie

## Patentansprüche

1. Endoskopkoppler (1) für eine Kamera mit einem proximalen Ende (2) des Endoskopkopplers (1) zur Anordnung an der Kamera, mit einem Kamerabefestigungsmechanismus (12a,12b) zur Befestigung des Endoskopkopplers (1) an der Kamera und mit einem Endoskopkopfaufnahmebereich (14) zur zumindest teilweisen Aufnahme eines Endoskopkopfes (90) wenn ein Endoskop mit der Kamera gekoppelt ist, wobei der Endoskopkopfaufnahmebereich (14) über einen Zugang (30) für den Endoskopkopf (90) aus Richtung eines distalen Endes (3) des Endoskopkopplers (1) zugänglich ist, und mit einem Endoskopfixiermechanismus zur Befestigung des Endoskops an dem Endoskopkoppler (1), wobei der Endoskopfixiermechanismus mindestens eine beweglich gelagerte Backe (15,16,17) aufweist, die mit einer Federkraft derart beaufschlagt ist, dass zumindest ein Bereich (115,116,117) der Backe (15,16,17) in den Zugang (30) hineingedrückt ist und den Zugang (30) sperrt, wobei zumindest der in den Zugang (30) hineinragende Bereich (115,116,117) der Backe (15,16,17) so geformt ist, dass sich sein distaler Abschnitt (126) in Richtung auf das distale Ende (3) des Endoskopkopplers (1) hin verjüngt, so dass ein Druck auf den distalen Abschnitt (126) des in den Zugang (30) hineinragenden Bereichs (115,116,117) der Backe (15,16,17) in Richtung auf das proximale Ende (2) des Endoskopkopplers (1) eine der Federkraft entgegenwirkende Kraft erzeugt und wobei der Endoskopkoppler (1) einen Lösemechanismus aufweist, bei dessen Betätigung in den Zugang (30) hineinragende Bereiche (115,116,117) der Backe (15,16,17) aus dem Zugang (30) herausbewegt werden oder aus dem Zugang (30) herausbewegbar werden,
**dadurch gekennzeichnet, dass** der Lösemechanismus derart aufgebaut ist, dass er bei Betätigung die Federkraftbeaufschlagung der Backe (15,16,17) reduziert.

2. Endoskopkoppler (1) nach Anspruch 1,
**dadurch gekennzeichnet, dass** der Lösemechanismus so aufgebaut ist, dass er durch eine Drehung in Umfangsrichtung des Endoskopopplers (1) ausgelöst wird.

3. Endoskopkoppler (1) nach einem der vorstehenden Ansprüche, **dadurch gekennzeichnet, dass** zumindest der in den Zugang (30) hineinragende Bereich (115,116,117) der Backe (15,16,17) so geformt ist, dass sich sein proximaler Abschnitt (127) in Richtung auf das proximale Ende (2) des Endoskopkopplers (1) hin schneller verjüngt als sich der distale Abschnitt (126) in Richtung auf das distale Ende (3) des Endoskopkopplers (1) hin verjüngt.

4. Endoskopkoppler (1) nach einem der vorstehenden Ansprüche, **dadurch gekennzeichnet, dass** die beweglich gelagerte Backe (15,16,17) auf ihrer einen Seite um eine parallel zur Einführrichtung des Endoskopkopfes (90) verlaufende Achse drehbar gelagert ist.

5. Endoskopkoppler (1) nach Anspruch 4,
**dadurch gekennzeichnet, dass** die um eine parallel zur Einführrichtung des Endoskopkopfes (90) verlaufende Achse drehbare Lagerung durch einen ersten Stift (21,22,23) erfolgt, der entweder nur in Richtung auf das distale Ende (3) des Endoskopkopplers (1) hin oder nur in Richtung auf das proximale Ende (2) des Endoskopkopplers (1) hin über die Backe (15,16,17) hinausragt.

6. Endoskopkoppler (1) nach Anspruch 4 oder 5,
**dadurch gekennzeichnet, dass** beweglich gelagerte Backe (15,16,17) auf ihrer anderen Seite in einer Führungsnut (27,28,29) verschiebbar gelagert ist.

7. Endoskopkoppler (1) nach Anspruch 5 und 6,
**dadurch gekennzeichnet, dass** die in der Führungsnut (27,28,29) verschiebbare Lagerung durch einen zweiten Stift (24,25,26) erfolgt, der nur in die der Richtung, in der der erste Stift (21,22,23), durch den die parallel zur Einführrichtung des Endoskopkopfes (90) verlaufende Achse drehbare Lagerung erfolgt, über die Backe (15,16,17) hinausragt, gegenüberliegende Richtung über die Backe (15,16,17) hinausragt.

8. Endoskopkoppler (1) nach Anspruch 6 oder 7,
**dadurch gekennzeichnet, dass** die Federkraftbeaufschlagung durch mindestens eine in der Führungsnut (27,28,29) angeordnete Feder (31) erfolgt.

9. Verfahren zur Kopplung eines Endoskops an eine Kamera unter Verwendung eines Endoskopkopplers (1) mit den Schritten
- Befestigen des Endoskopkopplers (1) an der Kamera und
- Befestigen des Endoskopkopfs (90) des Endoskops an dem Endoskopkoppler (1),
bei dem die Kopplung des Endoskopkopfes (90) an den Endoskopkoppler (1) lediglich durch Ausüben von Druck erfolgt, **dadurch gekennzeichnet, dass** als Endoskopkoppler (1) ein Endoskopkoppler (1) nach einem der Ansprüche 1 bis 8 verwendet wird.

10. Verfahren zur Entkopplung eines Endoskops von einem Endoskopkoppler (1), bei dem das Verfahren das Betätigen von genau einem Lösemechanismus umfasst, der auf sämtliche Fixiermittel, die das Endoskop, am Endoskopkoppler (1) halten, wirkt, **dadurch gekennzeichnet, dass** als Endoskopkoppler (1) ein Endoskopkoppler (1) nach einem der Ansprüche 1 bis 8 verwendet wird.

11. Verfahren zur Entkopplung eines Endoskops von einem Endoskopkoppler (1) nach Anspruch 10,
**dadurch gekennzeichnet, dass** der Lösemechanismus durch eine Drehbewegung in Umfangsrichtung des Endoskopkopplers (1) betätigt wird.

12. Kamera mit einem Endoskopkoppler (1) nach einem der Ansprüche 1 bis 8.

13. Kamera nach Anspruch 12,
**dadurch gekennzeichnet, dass** der Endoskopkoppler (1) mit der Kamera fest verbunden, insbesondere verpresst und/oder verschraubt ist.

14. Kamera nach Anspruch 12 oder 13,
**dadurch gekennzeichnet, dass** ein Teil des optischen Systems der Kamera am Endoskopkoppler (1) angeordnet ist.

## Claims

1. Endoscope coupler (1) for a camera, having a proximal end (2) of the endoscope coupler (1) for arranging on the camera, having a camera fixation mechanism (12a, 12b) for attaching the endoscope coupler (1) to the camera and having an endoscope head retention region (14) which at least partially holds an endoscope head (90) when an endoscope is coupled to the camera, wherein the endoscope head retention region (14) is accessible for the endoscope head (90) from the direction of a distal end (3) of the endoscope coupler (1) via an inlet (30), and having an endoscope fixation mechanism for attaching the endoscope to the endoscope coupler (1), wherein the endoscope fixation mechanism comprises at least one moveably mounted block (15, 16, 17) which is loaded with a spring tension such that at least one region (115, 116, 117) of the block (15, 16, 17) is pressed into the inlet (30), and blocks the inlet (30), wherein at least the region (115, 116, 117) of the block (15, 16, 17) which extends into the inlet (30) is formed such that its distal section (126) is tapered in the direction of the distal end (3) of the endoscope coupler (1) such that a pressure applied to the distal section (126) of the region (115, 116, 117) of the block (15, 16, 17) which extends into the inlet (30) in the direction of the proximal end (2) of the endoscope coupler (1) generates a force which counteracts the spring tension and wherein the endoscope coupler (1) comprises a release mechanism which, when actuated, moves the regions (115, 116, 117) of the block (15, 16, 17) which extend into the inlet (30) out of the inlet (30) or renders them moveable out of the inlet (30),
**characterized in that** the release mechanism is constructed such that, when actuated, it reduces the spring tension load of the block (15, 16, 17).

2. Endoscope coupler (1) in accordance with claim 1,
**characterized in that** the release mechanism is constructed such that it is actuated by means of a rotation of the endoscope coupler (1) in a circumferential direction.

3. Endoscope coupler (1) in accordance with either of the preceding claims, **characterized in that** at least the region (115, 116, 117) of the block (15, 16, 17) which extends into the inlet (30) is formed such that its proximal section (127) tapers more quickly in the direction of the proximal end (2) of the endoscope coupler (1) than the distal section (126) tapers in the direction of the distal end (3) of the endoscope coupler (1).

4. Endoscope coupler (1) in accordance with any of the preceding claims, **characterized in that** one side of the moveably mounted block (15, 16, 17) is rotatably mounted around an axis extending parallel to the insertion direction of the endoscope head (90).

5. Endoscope coupler (1) in accordance with claim 4,
**characterized in that** the mounting around an axis extending parallel to the insertion direction of the endoscope head (90) is carried out with a first pin (21, 22, 23), which either only protrudes over the block (15, 16, 17) in the direction of the distal end (3) of the endoscope coupler (1), or only in the direction of the proximal end (2) of the endoscope coupler (1).

6. Endoscope coupler (1) in accordance with claim 4 or 5,
**characterized in that** the other side of the moveably mounted block (15, 16, 17) is moveably mounted in a guide groove (27, 28, 29).

7. Endoscope coupler (1) in accordance with claims 5 and 6,
**characterized in that** the moveable mounting in the guide groove (27, 28, 29) is carried out with a second pin (24, 25, 26), which only protrudes over the block (15, 16, 17) in the direction opposite to the direction in which the rotatable mounting parallel to the insertion direction of the endoscope head (90) is carried out with the first pin (21, 22, 23).

8. Endoscope coupler (1) in accordance with claim 6 or 7,
**characterized in that** the loading with the spring tension is carried out by means of at least one spring (31) arranged in the guide groove (27, 28, 29).

9. Method for coupling an endoscope to a camera using an endoscope coupler (1) and having the steps
- attaching the endoscope coupler (1) to the camera and
- attaching the endoscope head (90) to the endoscope coupler (1),
in which the coupling of the endoscope head (90) to the endoscope coupler (1) is only carried out by means of pressure,
**characterized in that** an endoscope coupler (1) in accordance with any of claims 1 to 8 is used as endoscope coupler (1).

10. Method for decoupling an endoscope from an endoscope coupler (1), in which the method includes the activating of a release mechanism which functions with all means of fixation which hold the endoscope on the endoscope coupler (1), **characterized in that** an endoscope coupler (1) in accordance with any of claims 1 to 8 is used as endoscope coupler (1).

11. Method for decoupling an endoscope from an endoscope coupler (1) in accordance with claim 10,
**characterized in that** the release mechanism is activated by means of a rotational movement in circumferential direction of the endoscope coupler (1).

12. Camera having an endoscope coupler (1) in accordance with any of claims 1 to 8.

13. Camera in accordance with claim 12,
**characterized in that** the endoscope coupler (1) is fixedly attached to the camera, in particular by means of press-fitting and/or screwing.

14. Camera in accordance with claim 12 or 13,
**characterized in that** a part of the optical system of the camera is arranged on the endoscope coupler (1).

## Revendications

1. Coupleur d'endoscope (1) pour une caméra, avec une extrémité proximale (2) du coupleur d'endoscope (1) pour le montage à la caméra, un mécanisme de fixation de caméra (12a, 12b) pour fixer le coupleur d'endoscope (1) à la caméra et avec une zone de réception de tête d'endoscope (14) pour recevoir au moins en partie une tête d'endoscope (90) lorsqu'un endoscope est couplé à la caméra,
- la zone de réception de la tête d'endoscope (14) est accessible par un accès (30) pour la tête d'endoscope (90) dans la direction de l'extrémité distale (3) du coupleur d'endoscope (1) et avec un mécanisme de fixation d'endoscope pour fixer l'endoscope au coupleur d'endoscope (1),
- le mécanisme de fixation d'endoscope comprenant au moins un mors (15, 16, 17) monté mobile et sollicité par une force de ressort de façon qu'au moins une zone (115, 116, 117) du mors (15, 16, 17) soit enfoncée dans l'accès (30) et bloque l'accès (30),
- au moins la zone (115, 116, 117) du mors (15, 16, 17) qui vient en saillie dans l'accès (30) est formée pour que son segment distal (126) diminue en direction de l'extrémité distale (3) du coupleur d'endoscope (1), pour qu'une pression sur le segment distal (126) de la zone (115, 116, 117) du mors (15, 16, 17) qui vient en saillie dans l'accès (30), génère en direction de l'extrémité proximale (2) du coupleur d'endoscope (1), une force s'opposant à la force de ressort, et
- le coupleur d'endoscope (1) a un mécanisme de libération dont l'actionnement dégage la zone (115, 116, 117) du mors (15, 16, 17) en saillie dans l'accès (30), pour la dégager hors de l'accès (30) ou la sortir de l'accès (30),
coupleur **caractérisé en ce que**
le mécanisme de libération est construit pour lorsqu'il est actionné, réduire la force appliquée par le ressort du mors (15, 16, 17).

2. Coupleur d'endoscope (1) selon la revendication 1,
**caractérisé en ce que**
le mécanisme de libération est construit pour être libéré par une rotation dans la direction périphérique du coupleur d'endoscope (1).

3. Coupleur d'endoscope (1) selon l'une des revendications précédentes, **caractérisé en ce que**
au moins la zone (115, 116, 117) du mors (15, 16, 17) qui vient en saillie dans l'accès (30) est formée pour que son segment proximal (127) diminue plus rapidement en direction de l'extrémité proximale (2) du coupleur d'endoscope (1) que le segment distal (126) diminue en direction de l'extrémité distale (3) du coupleur d'endoscope (1).

4. Coupleur d'endoscope (1) selon l'une des revendications précédentes, **caractérisé en ce que**
le mors (15, 16, 17) monté mobile est monté à rotation sur son côté de façon rotative autour d'un axe parallèle à la direction d'engagement de la tête d'endoscope (90).

5. Coupleur d'endoscope (1) selon la revendication 4,
**caractérisé en ce que**
le montage à rotation autour d'un axe parallèle à la direction d'engagement de la tête d'endoscope (90) est réalisé par une première broche (21, 22, 23) qui dépasse par rapport au mors (15, 16, 17) soit seulement dans la direction de l'extrémité distale (3) du coupleur d'endoscope (1) soit dans la direction de l'extrémité proximale (2) du coupleur d'endoscope (1).

6. Coupleur d'endoscope (1) selon la revendication 4 ou 5,
**caractérisé en ce que**
le mors (15, 16, 17) monté mobile est monté coulissant par son autre côté dans une rainure de guidage (27, 28, 29).

7. Coupleur d'endoscope (1) selon la revendication 5 ou 6,
**caractérisé en ce que**
le montage coulissant dans la rainure de guidage (27, 28, 29) se fait par une seconde broche (24, 25, 26) qui ne dépasse du mors (15, 16, 17) que dans la direction par rapport au mors (15, 16, 17) opposée à la direction dans laquelle la première broche (21, 22, 23) réalise le montage à rotation autour d'un axe parallèle à la direction d'introduction de la tête d'endoscope (90).

8. Coupleur d'endoscope (1) selon la revendication 6 ou 7,
**caractérisé en ce que**
l'application de la force de ressort se fait par au moins un ressort (31) logé dans la rainure de guidage (27, 28, 29).

9. Procédé de couplage d'un endoscope à une caméra en utilisant un coupleur d'endoscope (1) avec les étapes consistant à :
- fixer le coupleur d'endoscope (1) à la caméra, et
- fixer la tête d'endoscope (90) au coupleur d'endoscope (1),
selon lequel
le couplage de la tête d'endoscope (90) au coupleur d'endoscope (1) se fait uniquement en exerçant une pression,
**caractérisé en ce que**
le coupleur d'endoscope (1) est un coupleur d'endoscope (1) selon l'une des revendications 1 à 8.

10. Procédé de découplage d'un endoscope par rapport à un coupleur d'endoscope (1) selon lequel le procédé d'actionnement comprend précisément un mécanisme de libération qui agit sur tous les moyens de fixation tenant l'endoscope au coupleur d'endoscope (1),
procédé **caractérisé en ce que**
le coupleur d'endoscope (1) est un coupleur d'endoscope (1) selon l'une des revendications 1 à 8.

11. Procédé de découplage d'un endoscope d'un coupleur d'endoscope (1) selon la revendication 10,
**caractérisé en ce que**
le mécanisme de libération est actionné par un mouvement de rotation dans la direction périphérique du coupleur d'endoscope (1).

12. Caméra comportant un coupleur d'endoscope (1) selon l'une des revendications 1 à 8.

13. Caméra selon la revendication 12,
**caractérisée en ce que**
le coupleur d'endoscope (1) est relié solidairement à la caméra, notamment par compression et/ou vissage.

14. Caméra selon la revendication 12 ou 13,
**caractérisée en ce que**
une partie du système optique de la caméra est sur le coupleur d'endoscope (1).
